# EUROPÄISCHE PATENTSCHRIFT

(11) **EP 0 629 144 B1**
(45) Veröffentlichungstag und Bekanntmachung des Hinweises auf die Patenterteilung: **18.10.1995**
(21) Anmeldenummer: 93906473.9
(22) Anmeldetag: 27.02.1993
(51) Int. Cl.: B01J 19/00, C07K 1/04

(54) **VORRICHTUNG ZUR DURCHFÜHRUNG ZEITGLEICH ODER SEQUENTIELL ABLAUFENDER CHEMISCHER REAKTIONEN**
DEVICE FOR SIMULTANEOUSLY OR SUCCESSIVELY CARRYING OUT CHEMICAL REACTIONS
DISPOSITIF POUR REALISER DES REACTIONS CHIMIQUES DE MANIERE SIMULTANEE OU SEQUENTIELLE

(30) Priorität: 02.03.1992 DE 4206488
(43) Veröffentlichungstag der Anmeldung: 21.12.1994
(73) Patentinhaber: DEUTSCHES KREBSFORSCHUNGSZENTRUM STIFTUNG DES ÖFFENTLICHEN RECHTS, 69009 Heidelberg (DE)
(72) Erfinder: DELIUS, Hajo, D-6915 Dossenheim (DE)
(74) Vertreter: Rückert, Friedrich, Dr.
(86) Internationale Anmeldenummer: EP9300457
(87) Internationale Veröffentlichungsnummer: WO9317785

(56) Entgegenhaltungen:
- WO-A-89/10188

## Beschreibung

Die Erfindung betrifft eine Vorrichtung zur Durchführung zeitgleich oder sequentiell ablaufender chemischer Reaktionen gemäß dem Oberbegriff des ersten Patentanspruchs.

Bei vielen chemischen Versuchsreihen ist es erforderlich, eine Vielzahl von Reaktionspartnern jeweils mit einer vorgegebenen Substanz zur Reaktion zu bringen und die jeweiligen Reaktionsprodukte zu verwerten, weiterzuverarbeiten oder zu analysieren. Bei anderen chemischen Versuchsreihen ist es erforderlich, eine vorgegebene Substanz sukzessiv mit einer Reihe von Reagentien in Kontakt zu bringen und die jeweiligen Reaktionsprodukte zu verwerten, weiterzuverarbeiten oder zu analysieren. Solche Versuchsreihen sind immer zeitaufwendig und stellen eintönige Routinearbeiten dar. Man versucht daher, solche Versuchsreihen mit Hilfe geeigneter Vorrichtungen oder Geräte zu automatisieren.

Insbesondere in der Biochemie müssen derartige Versuchsreihen, insbesondere der zweiten Art, häufig durchgeführt werden. Ein Beispiel hierfür ist die Oligonukleotid-Synthese, die eine grobe Bedeutung für eine Reihe von molekularbiologischen Techniken erlangt hat. Dabei sind insbesondere die Methoden der PCR (poly chain reaction) und die DNA-Sequenzanalyse zu erwähnen, bei denen sogenannte "Primer", kurze DNA-Einzelstränge mit einer Länge von ca. 16 bis 20 Nukleotiden zum Einsatz kommen.

Ein weiteres Problem besteht häufig darin, daß die Substanzen und ihre Reaktionspartner sehr teuer sind. Man wird daher versuchen, mit möglichst kleinen Mengen auszukommen.

Besonders in der Sequenz-Analyse mit der sogenannten "primer walking"-Methode, bei der jeweils die Primer-Sequenz für den nächsten Sequenzier-Schritt aus der neubestimmten Sequenz abgelesen und für die Synthese eines neuen Primers benützt wird, werden für die eigentliche Seguenzbestimmung nur sehr kleine Oligonukleotid-Mengen, etwa im pMol-Bereich, eingesetzt.

Es gibt eine Reihe von kommerziell erhältlichen Geräten, mit denen prinzipiell Synthesen in Versuchsreihen der ersten oder der zweiten Art und insbesondere Oligonukleotid-Synthesen durchgeführt werden können. Alle diese Geräte sind aber für Substanzmengen von mehr als 40 nMol ausgelegt, also für Mengen, die mehr als 1000 mal größer sind als benötigt. In vielen Fällen verhindert der hohe Preis von Primer-Synthesen mit den bekannten, kommerziell erhältlichen Geräten eine breite Anwendung dieser Methoden. Außerdem sind die kommerziell erhältlichen Geräte für maximal vier parallele Synthesen ausgelegt, so daß eine größere Zahl paralleler Synthesen, wie sie z. B. für Primer-Walking-Sequenzierung benötigt werden, nur in kleinen Arbeitsabschnitten, bei Neubeschickung des Geräts während der Arbeitszeit, jedoch z. B. nicht über Nacht durchgeführt werden kann.

Der Erfindung liegt daher die Aufgabe zugrunde, eine Vorrichtung vorzuschlagen, mit deren Hilfe eine im Prinzip nahezu unbegrenzte Zahl von chemischen Reaktionen zeitgleich oder sequentiell in der Art der oben erwähnten Versuchsreihen durchgeführt werden kann. Die Vorrichtung soll im Vergleich zu den bekannten Geräten mit geringeren Substanzmengen betrieben werden können. Ferner soll die Vorrichtung so konzipiert sein, daß sie automatisch gesteuert werden kann.

Die Aufgabe wird durch eine Vorrichtung mit den im ersten Patentanspruch genannten Merkmalen gelöst. Vorteilhafte Ausführungsformen der erfindungsgemäßen Vorrichtung sind in den abhängigen Ansprüchen angegeben.

Die Erfindung basiert auf den folgenden Überlegungen:
Wenn der Verbrauch von Reagentien niedrig gehalten werden soll, müssen sowohl das Volumen der Reaktionsräume als auch das Totvolumen zwischen Ventilen, über die die Reagentien zu den Reaktionsräumen geschaltet werden, und den Reaktionsräumen möglichst gering gehalten werden.

In den bekannten Geräten sind die Schaltventile über PTFE-Schläuche mit den Reaktionsräumen verbunden. Obwohl die Reaktionsräume nur ein Volumen von ca. 100 µl aufweisen, beträgt das gesamte freie Volumen ca. 200 µl.

Wenn weiterhin die Zeiten für eine Synthese auch für kleine Mengen kurz gehalten werden sollen, muß es möglich sein, die Konzentration der Reagentien trotz der kleinen benötigten Mengen hoch zu halten.

Beiden Forderungen steht die herkömmliche Anordnung von die üblichen Schaltventile und Reaktionsräumen entgegen.

Daher wird eine Vorrichtung vorgeschlagen, die ohne Ventile auskommt. Auf diese Weise kann das Totvolumen entscheidend vermindert werden.

In der Fig. 1 ist eine Ausführungsform der erfindungsgemäßen Vorrichtung dargestellt. Mit dieser Ausführungsform können gemäß der ersten Art der eingangs genannten Versuchsreihen vorgelegte Substanzen mit jeweils einem Reaktionspartner in Kontakt gebracht werden. Die Erfindung wird im folgenden anhand dieser Figur erläutert.

Fig. 2 stellt eine weitere Ausführungsform dar, die bei der oligonucleotid-Synthese eingesetzt wird.

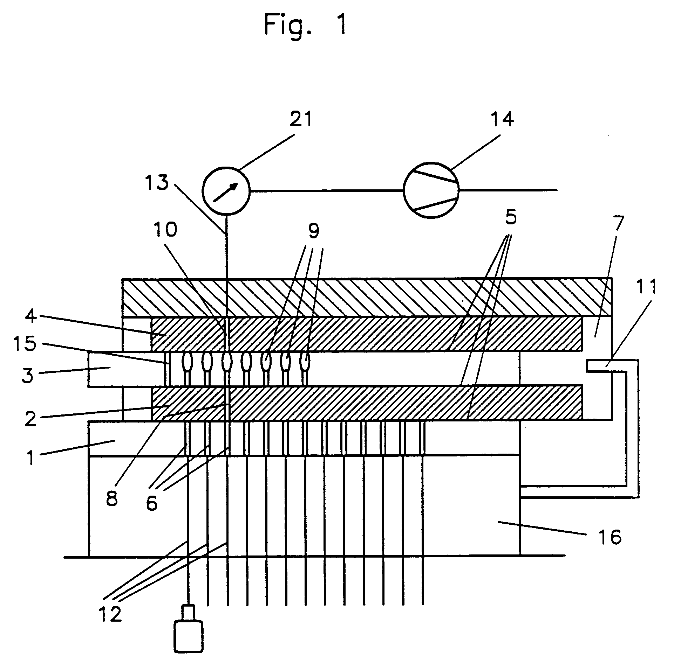

Die im folgenden verwendeten Begriffe "unten" und "oben" werden lediglich im Interesse einer klareren Darstellung der Erfindung verwendet und beziehen sich auf die in den Figuren dargestellten Ausführungsformen. Die z. B. in Fig. 1 dargestellte Vorrichtung kann jedoch auch umgedreht ("auf dem Kopf stehend") oder auf der Seite liegend betrieben werden. Die Erfindung umfaßt auch solche Ausführungformen.

Die erfindungsgemäße Vorrichtung besteht im wesentlichen aus vier Stäben 1, 2, 3, 4, die horizontal angeordnet sind und übereinander liegen. Die Stäbe berühren sich daher an sechs Flächen 5, die im folgenden als Kontaktflächen bezeichnet werden. Der obere und der untere Stab 4, 1 stehen über jeweils eine Kontaktfläche 5 mit dem benachbarten Stab in Berührung; die beiden mittleren Stäbe weisen jeweils zwei Kontaktflächen 5 auf. Die Kontaktflächen 5 sollen möglichst eben und glatt sein; sie sind z. B. durch Schleifen und Polieren in der Weise bearbeitet, daß sie spaltfrei gegeneinander bewegt werden können.

In der erfindungsgemäßen Vorrichtung sind die Kontaktflächen gegeneinander gepreßt. Der erforderliche Preßdruck kann durch Ausnutzung des Eigengewichts der Stäbe und/oder durch Federelemente aufgebracht werden. Die Höhe des Preßdrucks ist u. a. vom Betriebsdruck der Vorrichtung, der Viskosität der eingesetzten Reagentien und dem Material der Stäbe, insbesondere von der Oberflächenspannung der Reagentien auf dem Material, abhängig und wird in Abhängigkeit von diesen Parametern vorgegeben.

Mit dieser Anordnung wird erreicht, daß sich die Stäbe an ihren Kontaktflächen gegeneinander bewegen lassen und daß die Kontaktflächen dennoch eine Flüssigkeitssperre bilden und somit eine flüssigkeitsdichte Abdichtung bewirken.

Im übrigen ist die Form der Stäbe frei wählbar. Vorzugsweise wird man quaderförmige oder plattenförmige Stäbe verwenden. Die Stäbe können weiterhin an den nicht als Kontaktflächen verwendeten Seiten mit Führungsnuten oder -schienen, Federelementen und mit solchen Halteelementen versehen werden, die einen Austausch ermöglichen.

Der unterste, erste Stab 1 ist ortsfest mit der Vorrichtung verbunden. Beispielsweise kann der erste Stab auf einer Grundplatte 16 befestigt sein. Er weist ferner mehrere durchgehende Bohrungen 6 auf. Obwohl die Bohrungen prinzipiell nicht vertikal verlaufen müssen, wird man aus fertigungstechnischen Gründen die Bohrungen senkrecht zur Längsachse der Stäbe anordnen. Mit den Bohrungen können flüssigkeitsdicht, etwa über eingesetzte Fittings, von unten Reagentienleitungen 12 verbunden werden, die beispielsweise in Reagentien-Vorratsflaschen münden. Die Anzahl der Bohrungen 6 hängt von der Anzahl der für die Versuchsreihe notwendigen Reagentien ab. Man wird die Anzahl der Bohrungen so wählen, daß möglichst für jedes erforderliche Reagenz eine Bohrung und damit eine Reagentienleitung zur Verfügung steht, so daß die Vorratsflaschen während des Versuchs nicht gewechselt werden müssen.

Der über dem ersten Stab 1 liegende und mit diesem über die beiden jeweiligen Kontaktflächen 5 in Berührung stehende zweite Stab 2 ist fest mit einer Trägerplatte 7 verbunden und läßt sich gegenüber dem ersten Stab durch eine horizontale Bewegung der Trägerplatte verschieben. Dieser Stab weist eine durchgehende, vorzugsweise vertikal angebrachte Bohrung 8 auf.

Der über dem zweiten Stab 2 liegende und mit diesem über die beiden benachbarten Kontaktflächen in Berührung stehende dritte Stab 3 ist ebenfalls mit der Trägerplatte 7 verbunden, jedoch in der Weise, daß er relativ zu der Trägerplatte verschiebbar ist. Er weist eine Anzahl n von Reaktionsräumen 9 auf, die nach oben und nach unten offen sind. Die Form der Reaktionsräume richtet sich nach der Art der durchzuführenden Reaktion und nach der Menge der Reagentien. Prinzipiell können die Reaktionsräume durch Bohrungen hergestellt werden. Bei Oligonukleotid-Synthesen werden als Reaktionsräume häufig Kunststoff-Fritten eingesetzt, zwischen denen ein Träger aus porösem Glas gehalten wird. In diesem Fall kann es notwendig sein, daß die Reaktionsräume wie in der Figur angedeutet bauchig erweitert sind. Der dritte Stab 3 kann dann beispielweise aus zwei Schichten bestehen, von denen eine die Reaktionsräume und die unteren Öffnungen enthält und die andere als Deckel mit den entsprechenden oberen Öffnungen ausgebildet ist. Obwohl die oberen und die unteren Öffnungen der Reaktionsräume prinzipiell nicht vertikal übereinander angeordnet werden müssen, ist eine solche Anordnung aus fertigungstechnischen Gründen vorzuziehen. Die Anzahl n der Reaktionsräume hängt von der Anzahl der durchzuführenden Reaktionen ab und ist - bei entsprechender Länge der Stäbe - im Prinzip frei wählbar.

Es erweist sich als vorteilhaft, wenn alle Stäbe, zumindest jedoch der dritte Stab 3 temperierbar und thermostatisierbar sind. In diesem Fall können Reaktionen auch bei vorgegebener Temperatur oberhalb oder unterhalb der Raumtemperatur durchgeführt werden.

Der über dem dritten Stab 3 liegende und mit diesem über die beiden benachbarten Kontaktflächen in Berührung stehende vierte Stab 4 ist ebenso wie der zweite Stab 2 fest mit der Trägerplatte 7 verbunden und enthält eine durchgehende Bohrung 10. Für diese Bohrung wird wiederum eine vertikale Anordnung bevorzugt.

Für die Funktion der Vorrichtung ist es wesentlich, daß es n verschiedene Stellungen der Stäbe 2, 3 und 4 gibt, bei denen die Bohrungen 8 und 10 sowie die obere und die untere Öffnung eines der Reaktionsräume eine durchgehende Verbindung darstellen. Die Anzahl n entspricht dabei der Anzahl der vorgesehenen Reaktionsräume. Diese Bedingung ist am einfachsten erfüllt, wenn die Bohrungen 8 und 10 senkrecht ausgeführt wurden, auf einer gemeinsamen Geraden liegen und wenn die obere und die untere Öffnung eines jeden Reaktionsraumes senkrecht übereinander angeordnet sind. Prinzipiell können jedoch die Bohrungen 8 und 10 auch in anderen Winkeln sowie die obere und die untere Öffnung eines jeden Reaktionsraums gegeneinander verschoben angebracht werden, solange die genannte Bedingung erfüllt ist.

Die Bohrung 10 des vierten Stabs 4 kann z. B. über geeignete Fittings mit einer Leitung 13 verbunden sein, in die eine Pumpe 14 eingesetzt ist. Eine Pumpe ist insbesondere dann erforderlich, wenn die Reagentienleitungen 12, die in den unteren Teil der Bohrungen 6 münden, und die hieran angeschlossenen Reagentien-Vorratsflaschen nicht in der Weise angeordnet sind, daß die Reagentien aufgrund eines Gefälles in die Vorrichtung strömen. Weiterhin kann auf eine Pumpe verzichtet werden, wenn die Vorrichtung gegenüber der in der Figur dargestellten Ausführungsform "aufdem Kopf stehend" betrieben wird, so daß der erste Stab 1 den oberen und der vierte Stab 4 den unteren Stab darstellt. In diesen Fällen kann für manche Verwendungszwecke der erfindungsgemäßen Vorrichtung auf eine Pumpe verzichtet werden.

Für die in der Figur dargestellte Ausfünrungsform wird jedoch im allgemeinen eine Saugpumpe eingesetzt werden. Besonders bevorzugt wird eine solche Saugpumpe, bei der die Förderrichtung umgekehrt werden kann, z. B. eine Schlauchpumpe. Durch eine zeitweise Umkehrung der Förderrichtung kann eine gute Durchmischung der Reagentien in den Reaktionsräumen 9 erreicht werden. Zusätzlich zu der Pumpe 14 kann auch ein Durchflußmonitor 21 in die Leitung integriert werden.

Für einen automatisierten Betrieb der erfindungsgemäßen Vorrichtung ist es äußerst vorteilhaft, wenn sowohl die Bohrungen 6 im ersten Stab 1 als auch die oberen und unteren Öffnungen der Reaktionsräume 9 im dritten Stab 3 in einem konstanten Abstand angebracht sind. In diesem Fall kann die Trägerplatte 7 durch einen automatisch, z. B. durch einen entsprechend programmierten Computer, gesteuerten Schrittmotor horizontal bewegt werden, wobei die Schritte des Schrittmotors dem konstanten Abstand entsprechen und die jeweilige Zeitdauer einer Position durch den Computer vorgegeben wird. Über einen Schrittmotor und den Computer kann auch die Pumpe angesteuert werden.

Der dritte Stab 3, der die Reaktionsräume 9 enthält, wird beim Betrieb der Vorrichtung relativ zu der Trägerplatte 7 bewegt. Diese Bewegung kann durch verschiedene Konstruktionselemente erzielt werden. Beispielsweise ein weiterer Schrittmotor für die horizontale Bewegung des dritten Stabs 3 vorgesehen werden. Auch dieser Schrittmotor kann läßt sich durch einen Computer steuern. Häufig erscheint es jedoch einfacher, einen Anschlag 11 oder zwei Anschläge 11, 11' vorzusehen, der bei sich bewegender Trägerplatte 7 dafür sorgt, daß der dritte Stab 3 relativ zur sich bewegenden Trägerplatte 7 zumindest zeitweise in einer ortsfesten Stellung gehalten wird. Stößt der dritte Stab 3 nicht an den Anschlag an, wird er wegen der Reibung der Kontaktflächen zusammen mit der Trägerplatte bewegt.

Auch der Anschlag kann prinzipiell z. B. mechanisch über ein Schraubgewinde oder automatisch über einen Motor und einen Computer verstellbar konstruiert sein. In welcher Weise der Anschlag 11 angebracht ist, hängt davon ab, ob die erste oder die zweite Art der eingangs erwähnten Versuchsreihen durchgeführt werden soll.

In Fig. 1 ist der Anschlag auf der rechten Seite der Figur angebracht und bewirkt, daß die Reaktionsräume 9 des dritten Stabs 3 bei sich nach rechts bewegender Trägerplatte 7 ortsfest relativ zum ersten Stab 1 und zu der Grundplatte gehalten wird. Auf diese Weise lassen sich die Reaktionsräume 9 nacheinander zwischen den Bohrungen 8 und 10 in den Stäben 2 bzw. 4 positionieren, um nach dem Verschieben der Trägerplatte 7 nach links über die Reagentienleitungen 12 einen Reagentienfluß durch die ausgewählte Reaktionskammer zu ermöglichen.

Der dritte Stab 3 enthält außer den Reaktionsräumen 9 vorzugsweise eine Bohrung 15, die seitlich neben der Gruppe der Reaktionsräume angebracht ist, wobei man die Bohrung 15 in der Weise anordnen wird, daß ihr Abstand zu den oberen und unteren Öffnungen des nächstliegenden Reaktionsraumes dem oben erwähnten konstanten Abstand entspricht. Mit dieser Bohrung 15 kann die Vorrichtung gespült werden. Ferner lassen sich über diese Bohrung die Leitungen 12 und 13 entlüften.

Der Durchmesser sämtlicher Bohrungen sollte auf Art der Reagentien, die zu verwendenden Synthesemengen und auf den erwünschten Durchfluß, somit auf den tolerierbaren Stömungswiderstand abgestimmt werden, weil das Totvolumen mit dem Durchmesser ansteigt. Prinzipiell existieren jedoch keine Einschränkungen bezüglich des Durchmessers der Bohrungen.

Für die Stäbe der erfindungsgemäßen Vorrichtung und insbesondere für die Kontaktflächen 5 können prinzipiell alle Materialien verwendet werden, die sich maßgenau pressen oder spanend bearbeiten, somit bohren, fräsen und schleifen lassen. Die Stäbe lassen sich gegebenenfalls auch aus Kunststoff, z. B. durch Spritzguß herstellen. Einschränkungen können sich hinsichtlich der einzusetzenden Reagentien ergeben, gegen die zumindest die Bohrungen 6, 8, 10, die Reaktionsräume 9 und die Kontaktflächen 5 beständig sein müssen. Für die Stäbe kann z. B. ein Edelstahl verwendet werden, wobei man die Kontaktflächen gegebenenfalls besonders behandeln oder beschichten wird. Ein sehr geeigneter Werkstoff für die Stäbe ist sogenanntes "machinable glass", eine Mischung aus Glas und Glimmer, das sich mit hoher Präzision sägen, fräsen, bohren und schleifen läßt. Dieser Werkstoff, der gegen die meisten aggressiven Reagentien beständig ist, wird z. B. unter dem Warenzeichen MACOR von der Firma Corning, USA vertrieben.

Die Stäbe brauchen nicht aus demselben Material zu bestehen. Beispielsweise kann ein dritter Stab aus Stahl oder Aluminium mit MACOR^{R}-Stäben kombiniert werden.

Die erfindungsgemäße Vorrichtung weist gegenüber den bekannten Vorrichtungen eine Reihe von wesentlichen Vorzügen auf.

Die Dichtigkeit geschliffener Kontaktflächen zwischen den Stäben ist im allgemeinen völlig ausreichend für das Ansaugen der Reagentien auch aus tiefer gelegenen Reagentien-Vorratsflaschen und gut genug, um Kreuzkontaminationen der Reagentien hinreichend klein zu halten. Werden die Kontaktflächen frei von Kratzern gehalten, ist zu erwarten, daß die Dauerhaftigkeit der erfindungsgemäßen Vorrichtung besser ist als die Dauerhaftigkeit herkömmlicher PTFE/Stahlventile in den bekannten Vorrichtungen.

Ein weiterer Vorteil ist, daß das Totvolumen sehr klein, z. B. bei ca 10 µl, gehalten werden kann.

Die Reagentienräume können mit sehr geringen Volumina von Reagentien gespült werden und die Reagentien werden nur mit geringer Vermischung an die Reagentienräume herangeführt. Der Verbrauch an Reagentien kann deshalb optimal an kleine Synthesemengen angepaßt werden. Beispielsweise konnten mit einem Prototyp der erfindungsgemäßen Vorrichtung Oligonukleotid-Synthesen mit Trägermaterial für ca 10 nMol mit einem Gesamtdurchsatz an Reagentien von nur 1,7 ml pro Kopplung durchgeführt werden; dies ist ca 4 bis 5 mal weniger als bei bekannten Geräten.

Die erfindungsgemäße Vorrichtung kann zudem in vielfältiger Form an die zu lösenden Aufgaben angepaßt werden. So ist die Zahl der Reagentienräume und Reagentienleitungen bei entsprechend langen Stäben nahezu frei wählbar. Die Reagentienräume und die Bohrungen für die Reagentienleitungen können zudem in parallelen Reihen angeordnet werden, wobei die Trägerplatte horizontal nicht nur in einer Richtung (x-Achse), sondern in zwei Richtungen (x- und y-Achse bewegt wird. In diesem Fall kann es vorteilhaft sein, wenn der zweite 2 und der vierte 4 Stab nicht nur eine, sondern mehrere Bohrungen enthält, die über den parallelen Reihen der Reagentienräume und der Bohrungen für die Reagentienräume liegen.

Die Erfindung und ihre Wirkungweise wird im folgenden anhand eines Durchführungsbeispiels näher erläutert.

### Beispiel

### Ausführungsform für die Oligonukleotid-Synthese

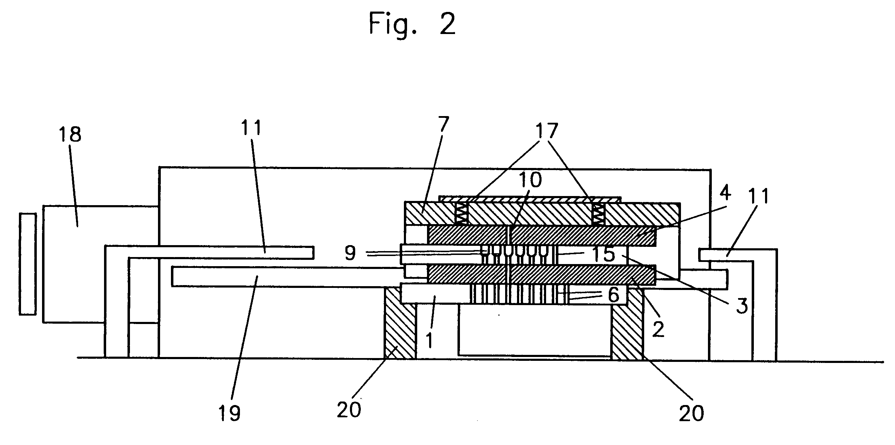

Die für die Oligonukleotid-Synthese eingesetzte Ausführungsform ist in Fig. 2 dargestellt; sie enthielt 7 Reagentienräume 9 und 14 Bohrungen 6 im ersten Stab, die jeweils in einer einzigen Reihe angeordet waren. Sämtliche Bohrungen verliefen senkrecht. Die Trägerplatte wurde über eine Spindel 19 durch einen Schrittmotor 18 bewegt, der über einen Computer gesteuert wurde. In die Leitung 13 an Bohrung 10 war eine Schlauchpumpe 14 eingesetzt, die ebenfalls durch den Computer gesteuert wurde. Die Effizienz der Synthese wurde durch einen Absorptions-Durchfluß-Monitor überwacht, der zwischen Bohrung 10 und die Schlauchpumpe 14 in die Leitung 13 eingesetzt wurde.

Die Vorrichtung enthielt ferner zwei Anschläge 11, 11'. Mit den Blöcken 20 wurde der Stab 1 auf der Grundplatte fixiert. Diese Blöcke sind vor der verschiebbaren Trägerplatte 7 montiert.

### Durchführung einer Oligonukleotid-Synthese

Mit der in Beispiel 1 beschriebenen Ausführungsform der erfindungsgemäßen Vorrichtung wurden Oligonukleotid-Synthesen durchgeführt.
Die einzelnen Nukleotid-Bausteine wurden in sich wiederholenden Synthesezyklen an das erste aktivierte Nukleotid angekoppelt, welches an dem porösen Glas in einem Reaktionsraum zwischen den Kunststoff-Fritten gehalten wurde. Diese Zyklen bestehen aus
a) Entfernen der Trityl-Schutzgruppe durch Trichloressigsäure in Dichlorethan gelöst,
b) Ankopplung des Nukleotid-Bausteins, der als Phosphoramidit in stabilisierter Form in Acetonitril gelöst vorlag,
c) Blockierung der Nukleotid-Enden, die nicht reagiert haben, durch das sogenannte Capping,
d) Oxidation des Phosphors durch Jod.

Vorraussetzung für eine Synthese der Oligonukleotide mit guter Effizienz ist ein möglichst vollständiger Ausschluß von Feuchtigkeit. Dieser konnte durch die eingesetzte Vorrichtung gewährleistet werden.

### Vorbereitung einer Synthese:

Die Federn 17 werden gelöst, so daß Stab 4 abgehoben werden und Stab 3 dem Gerät entnommen werden kann. In die Reaktionsräume 9 wird die Säulenfüllung eingebracht. Dazu wird jeweils eine Fritte (3,0 mm x 1,5 mm, Polyethylen) in eine Reaktionskammer 9 gedrückt, gefolgt von ca. 2 - 3 mg eines Nukleosid-beladenen CPG-Trägers (Millipore, ca. 5 µmol/g, 500 Angström Poren) und einer zweiten Fritte als Abdeckung. Der Nukleosidträger wird entsprechend der zu synthetisierenden Sequenz für die entsprechende Reaktionskammer ausgewählt (G, A, T oder C).

Nach Einschalten des Gerätes wird die Trägerplatte 7 nach links in eine Referenzposition gefahren. Der Stab 3, der mit den Säulenfüllungen beladen ist, wird wieder in das Gerät eingesetzt. Der Stab 4 wird wieder aufgelegt und die Federn gespannt, um beide Stäbe, 3 und 4, an den Stab 2 zu pressen. Der Druck beträgt im Prototyp ca. 500 p, richtet sich aber nach der Qualität der Kontaktflächen.

Die Trägerplatte wird unter PC-Kontrolle nach rechts gefahren, so daß der rechte Anschlag 11' den Stab 3 so weit verschiebt, daß Bohrung 15 in Stab 3 mit Bohrung 8 in Stab 2 und Bohrung 10 in Stab 4 fluchtet.

Danach kann die Trägerplatte wieder nach links verschoben werden, so daß Bohrung 8 in Stab 2 über die jeweils benötigte Reagentienleitung 6 zu stehen kommt.

Zur Entlüftung und Spülung der Reagentienleitungen werden nun vorbestimmte Mengen aller Reagentien durch die Bohrungen 8, 15 und 10 über die Leitung 13 und die Pumpe 14 abgepumpt.

Die Reihenfolge ist:
Dichlorethan, Trichloressigsäure (TCA, 1 % in Dichlorethan), Dichlorethan, Acetonitril, Capping-Reagenz A, Capping Reagenz B, Oxidations-Reagenz, Acetronitril, Tetrazol, A-Amidit, T-Amidit, C-Amidit und G-Amidit, Acetronitril, Dichlorethan.

Danach ist das Gerät bereit zur Oligonukleotidsynthese.

Die zu synthetisierenden Sequenzen werden über die Tastatur am PC eingegeben. Dabei können am Prototyp bis zu sieben Sequenzen von einer Länge bis zu 50 Nukleotiden eingegeben werden.

Nach der Eingabe wird die Synthese vom PC aus startet. Zur Synthese des ersten Oligonuklotids wird die Trägerplatte so weit nach links gefahren, daß der Stab 3 zwischen den Stäben 2 und 4 durch den linken Anschlag nach rechts verschoben wird, bis die erste Reaktionskammer 9 in Stab 3 mit den Bohrungen 8 und 10 in den Stäben 2 und 4 fluchtet.

### Beginn einer Synthese

Zum Beginn der Synthese wird zunächst die Trägerplatte wieder nach rechts verschoben, bis die Bohrung 8 über der Reaktionsleitung für Dichlorethan steht. Die Reaktionskammer 9, welche die Säulenfüllung enthält, wird mit 140 µl Dichlorethan gewaschen. Dabei wird im Waschprogramm durch eine kurze Pump-Phase in umgekehrter Richtung für eine gute Durchmischung gesorgt.

Danach wird die Trägerplatte in die TCA-Position gefahren. Die Reaktionskammer wird mit TCA durchspült, wodurch die Schutzgruppe am Nukleotid des CPG-Materials entfernt wird. Danach wird wieder mit Dichlorethan gewaschen.

### Addition eines Nukleotids

1. Waschen mit Acetonitril. Wie beim Waschen mit Dichlorethan: 140 µl mit kurzer Pumpumkehr.
2. Zugabe von Amidit-Lösung und Tetrazol, dem Aktivator. Es werden zur besseren Durchmischung dreimal im Wechsel 10 µl Tetrazol und 10 µl Amiditlösung in die Reaktionskammer gepumpt, gefolgt von 10 µl Tetrazol. Zur Reaktion wird die Trägerplatte in die Acetonitril-Position gefahren. In dieser Stellung wird zur weiteren Durchmischung während der Reaktion mit langsamer Pumpgeschwindigkeit das Reagenzgemisch in der Reaktionskammer dreimal um 10 µl zurück- und vorgepumpt. Nach Abschluß der Reaktion wird mit Acetonitril gewaschen.
3. Zum Inaktivieren der freien Gruppen, die kein Amidit angekoppelt haben, werden die Reagenzien Capping A und Capping B in die Reaktionskammer gepumpt und kurz vermischt.
4. Nach kurzem Waschen mit Acetonitril wird Oxidations-Reagenz durch die Kammer gepumpt.
5. Nach nochmaligem Waschen mit Acetonitril, gefolgt von Dichlorethan wird mit TCA die Trityl-Schutzgruppe von dem neu angefügten Amidit abgespalten. Die Menge dieses Tritylrestes kann im Durchfluß-Monitor durch Absorptionsmessung bei einer Wellenlänge von 550 nm bestimmt werden.

Mit diesem Schritt ist die Ankoppelung eines Nukleotids abgeschlossen. Wenn ein weiteres Nukleotid angefügt werden soll, wird wie in den Schritten 1 bis 5 verfahren.

Nach Ankoppelung des letzten Nukleotids der eingegebenen Oligonukleotid-Sequenz wird mit Dichlorethan gewaschen.

Falls ein weiteres Oligonukleotid synthetisiert werden soll, wird die Trägerplatte nach links verschoben, bis der Stab 3 durch den linken Anschlag so weit nach rechts verschoben ist, daß die nächste Reaktionskammer mit den Bohrungen 8 und 10 in den Stäben 2 und 4 fluchtet. Dann wird die Synthese des nächsten Oligonukleotids wie oben beschrieben durchgeführt.

## Patentansprüche

1. Vorrichtung zur Durchführung zeitgleich oder sequentiell ablaufender chemischer Reaktionen mit den Merkmalen:
a) vier horizontal angeordnete Stäbe (1, 2, 3, 4) sind übereinander liegend angeordnet;
b) die äußeren Stäbe (1, 4) weisen je eine, die mittleren Stäbe (2, 3) weisen je zwei einander gegenüberliegende Kontaktflächen (5) auf, über die sie untereinander in Kontakt stehen;
c) die Kontaktflächen (5) sind in einer Weise bearbeitet und gegeneinander gepreßt, daß
- die Stäbe horizontal gegeneinander verschiebbar sind,
- die Kontaktflächen eine flüssigkeitsdichte Abdichtung bewirken;
d) der erste Stab (1)
- weist mehrere durchgehende Bohrungen (6) auf und
- ist mit der Vorrichtung fest verbunden;
e) der mit dem ersten Stab (1) in Kontakt stehende zweite Stab (2) ist
- fest mit einer relativ zum ersten Stab (1) horizontal bewegbaren Trägerplatte (7) verbunden und
- weist eine durchgehende Bohrung (8) auf;
f) der mit dem zweiten Stab (2) in Kontakt stehende dritte Stab (3) ist
- horizontal verschiebbar auf der Trägerplatte (7) angebracht und
- weist eine Anzahl n von oben und unten mit Öffnungen versehenen, Reaktionsräumen (9) auf;
g) der mit dem dritten Stab (3) in Kontakt stehende vierte Stab (4) ist
- fest mit der Trägerplatte (7) verbunden und
- weist eine durchgehende Bohrung (10) auf;
h) es sind n verschiedene Stellungen des dritten Stabs (3) relativ zum zweiten (2) und vierten (4) Stab einstellbar, bei denen die Bohrungen (8, 10) und die Öffnungen eines der Reaktionsräume (9) eine durchgehende Verbindung herstellen.

2. Vorrichtung nach Anspruch 1, dadurch gekennzeichnet, daß die Bohrungen (8,10) des zweiten (2) und des vierten (4) Stabes vertikal verlaufen und auf einer gemeinsamen Geraden liegen.

3. Vorrichtung nach Anspruch 1 oder 2, dadurch gekennzeichnet, daß die Bohrungen (6) des ersten Stabs (1) flüssigkeitsdicht mit Reagentienleitungen (12) verbunden sind.

4. Vorrichtung nach Anspruch 1 oder 2, dadurch gekennzeichnet, daß die Bohrung (10) des vierten Stabs (4) flüssigkeitsdicht mit einer Leitung (13) verbunden ist.

5. Vorrichtung nach Anspruch 4, dadurch gekennzeichnet, daß in die Leitung (13) eine Pumpe (14) eingesetzt ist.

6. Vorrichtung nach einem der Ansprüche 1 bis 5, dadurch gekennzeichnet, daß die Bohrungen (6) im ersten Stab (1) und die oberen und unteren Öffnungen der Reaktionsräume (9) im dritten Stab (3) jeweils in einem konstanten Abstand angebracht sind.

7. Vorrichtung nach Anspruch 1 oder 6, dadurch gekennzeichnet, daß der dritte Stab (3) durch ein Konstruktionselement in der Weise gegenüber der Trägerplatte (7) horizontal verschoben wird, daß die Bohrungen (8) und (10) des zweiten (2) und vierten (4) Stabs mit der oberen und der unteren Öffnung eines der Reaktionsräume (9) fluchten.

8. Vorrichtung nach Anspruch 7, dadurch gekennzeichnet, daß das Konstruktionselement ein Anschlag ist.

9. Vorrichtung nach Anspruch 7, dadurch gekennzeichnet, daß das Konstruktionselement ein erster Schrittmotor ist.

10. Vorrichtung nach einem der Ansprüche 1 bis 9, dadurch gekennzeichnet, daß die Trägerplatte (7) durch einen zweiten Schrittmotor horizontal bewegt wird.

11. Vorrichtung nach Anspruch 9, dadurch gekennzeichnet, daß der erste Schrittmotor von einem Computer gesteuert ist.

12. Vorrichtung nach Anspruch 10, dadurch gekennzeichnet, daS der zweite Schrittmotor von einem Computer gesteuert ist.

13. Vorrichtung nach Anspruch 5, dadurch gekennzeichnet, daß die Pumpe (14) von einem Computer gesteuert ist.

14. Vorrichtung nach Anspruch 6, dadurch gekennzeichnet, daß der dritte Stab (3) eine durchgehende Bohrung (15) enthält, die in dem konstanten Abstand zu der oberen und unteren Öffnung eines der Reaktionsräume (9) angebracht ist.

15. Vorrichtung nach Anspruch 1, dadurch gekennzeichnet, daß die Stäbe (1, 2, 3, 4) aus einem spanend bearbeitbaren Glas bestehen.

## Claims

1. Apparatus for effecting simultaneously or sequentially occurring chemical reactions, having the features:
a) four horizontally disposed bars (1, 2, 3, 4) are disposed above one another;
b) the outer bars (1, 4) each have a contact face (5), and the central bars (2, 3) each have two oppositely situated contact faces (5), via which they are in contact with one another;
c) the contact faces (5) are processed and pressed towards one another in such a manner that
- the bars are displaceable horizontally relative to one another,
- the contact faces effect a fluid-tight sealing;
d) the first bar (1)
- has a plurality of continuous bores (6) and
- is firmly connected to the apparatus;
e) the second bar (2), which is in contact with the first bar (1),
- is firmly connected to a carrier plate (7), which is horizontally displaceable relative to the first bar (1), and
- has a continuous bore (8);
f) the third bar (3), which is in contact with the second bar (2),
- is horizontally displaceably mounted on the carrier plate (7) and
- has a number n of reaction chambers (9), which are provided with apertures at its upper and lower ends;
g) the fourth bar (4), which is in contact with the third bar (3),
- is firmly connected to the carrier plate (7), and
- has a continuous bore (10);
h n different positions of the third bar (3) can be set relative to the second (2) and fourth (4) bars, wherein the bores (8, 10) and the apertures of one of the reaction chambers (9) establish a continuous connection.

2. Apparatus according to claim 1, characterised in that the bores (8, 10) of the second (2) and fourth (4) bars extend vertically and lie on a common straight line.

3. Apparatus according to claim 1 or 2, characterised in that the bores (6) of the first bar (1) are connected in a fluid-tight manner to reagent lines (12).

4. Apparatus according to claim 1 or 2, characterised in that the bore (10) of the fourth bar (4) is connected in a fluid-tight manner to a line (13).

5. Apparatus according to claim 4, characterised in that a pump (14) is inserted in the line (13).

6. Apparatus according to one of claims 1 to 5, characterised in that the bores (6) in the first bar (1) and the upper and lower apertures of the reaction chambers (9) in the third bar (3) are each provided at a constant spacing.

7. Apparatus according to claim 1 or 6, characterised in that the third bar (3) is displaced horizontally relative to the carrier plate (7) by a structural element in such a manner that the bores (8) and (10) of the second (2) and fourth (4) bars are in alignment with the upper and lower apertures of one of the reaction chambers (9).

8. Apparatus according to claim 7, characterised in that the structural element is a stop member.

9. Apparatus according to claim 7, characterised in that the structural element is a first stepping motor.

10. Apparatus according to one of claims 1 to 9, characterised in that the carrier plate (7) is horizontally displaced by a second stepping motor.

11. Apparatus according to claim 9, characterised in that the first stepping motor is controlled by a computer.

12. Apparatus according to claim 10, characterised in that the second stepping motor is controlled by a computer.

13. Apparatus according to claim 5, characterised in that the pump (14) is controlled by a computer.

14. Apparatus according to claim 6, characterised in that the third bar (3) contains a continuous bore (15), which is provided at a constant spacing relative to the upper and lower apertures of one of the reaction chambers (9).

15. Apparatus according to claim 1, characterised in that the bars (1, 2, 3, 4) are formed from a machinable glass.

## Revendications

1. Dispositif pour réaliser des réactions chimiques de manière simultanée ou séquentielle, ayant les caractéristiques suivantes :
a) quatre tiges horizontales (1, 2, 3, 4) superposées,
b) les tiges extérieures (1, 4) ont chacune une surface de contact et les tiges intermédiaires (2, 3) ont chacune deux surfaces de contact (5) par lesquelles les différentes tiges sont en contact,
c) les surfaces de contact (5) sont usinées et pressées les unes sur les autres pour que,
- les tiges horizontales puissent coulisser les unes par rapport aux autres,
- les surfaces de contact réalisent une étanchéité vis-à-vis des liquides,
d) la première tige (1),
- comporte plusieurs perçages traversants (6),
- est reliée solidairement au dispositif,
e) la seconde tige (2) en contact avec la première tige (1) est,
- reliée solidairement à une plaque de support (7) mobile horizontalement par rapport à la première tige (1) et,
- comporte un perçage traversant (8) ,
f) la troisième tige (3) en contact avec la seconde tige (2),
- est coulissante horizontalement sur la plaque de support (7) et,
- comporte un nombre n de chambres de réaction (9) munies d'ouvertures vers le haut et vers le bas,
g) la quatrième tige (4) en contact avec la troisième tige (3) est,
- reliée solidairement à la plaque de support (7),
- comportant un perçage traversant (10),
h) il y a n positions différentes pour la troisième tige (3) par rapport à la seconde tige (2) et la quatrième tige (4), pour lesquelles les perçages (8, 10) et les ouvertures d'une chambre de réaction (9) réalisent une liaison continue.

2. Dispositif selon la revendication 1, caractérisé en ce que les perçages (8, 10) de la seconde tige (2) et de la quatrième tige (4) sont verticaux et se situent sur une droite commune.

3. Dispositif selon la revendication 1 ou 2, caractérisé en ce que les perçages (6) de la première tige sont reliés de manière étanche au liquide à des conduites de réactifs (12).

4. Dispositif selon la revendication 1 ou 2, caractérisé en ce que le perçage (10) de la quatrième tige (4) est relié à la conduite (13) d'une manière étanche au liquide.

5. Dispositif selon la revendication 4, caractérisé en ce que la conduite (13) est munie d'une pompe (14).

6. Dispositif selon l'une des revendications 1 à 5, caractérisé en ce que les perçages (6) de la première tige (1) et les ouvertures supérieures et inférieures des chambres de réaction (9) de la troisième tige (3) sont prévues suivant un intervalle constant.

7. Dispositif selon la revendication 1 ou 6, caractérisé en ce que la troisième tige (3) peut être coulissée horizontalement par rapport à la plaque de support (7) par un élément de construction de façon que les perçages (8 et 10) de la seconde tige (2) et de la quatrième tige (4) sont alignés sur l'ouverture supérieure et l'ouverture inférieure d'une des chambres de réaction (9).

8. Dispositif selon la revendication 7, caractérisé en ce que l'élément de construction est une butée.

9. Dispositif selon la revendication 7, caractérisé en ce que l'élément de construction est un premier moteur pas à pas.

10. Dispositif selon l'une des revendications 1 à 9, caractérisé en ce que la plaque de support (7) est déplacée horizontalement par un second moteur pas à pas.

11. Dispositif selon la revendication 10, caractérisé en ce que le premier moteur pas à pas est commandé par un ordinateur.

12. Dispositif selon la revendication 10, caractérisé en ce que le second moteur pas à pas est commandé par un ordinateur.

13. Dispositif selon la revendication 5, caractérisé en ce que la pompe (14) est commandée par un ordinateur.

14. Dispositif selon la revendication 6, caractérisé en ce que la troisième tige (3) comporte un perçage traversant (15) qui se trouve à l'intervalle constant par rapport à l'ouverture supérieure à l'ouverture inférieure de l'une des chambres de réaction (9).

15. Dispositif selon la revendication 1, caractérisé en ce que les tiges (1, 2, 3, 4) sont réalisées en verre susceptible d'être usiné.
